# EUROPÄISCHE PATENTSCHRIFT

(11) **EP 0 927 158 B1**
(45) Veröffentlichungstag und Bekanntmachung des Hinweises auf die Patenterteilung: **31.10.2001**
(21) Anmeldenummer: 97933692.2
(22) Anmeldetag: 19.07.1997
(51) Int. Cl.: C07C 309/17, C07C 309/19, C07C 309/22, C07C 309/24, C11D 1/28, B01F 17/00, A61K 7/50

(54) **AMPHIPHILE VERBINDUNGEN MIT MINDESTENS ZWEI HYDROPHILEN UND MINDESTENS ZWEI HYDROPHOBEN GRUPPEN AUF DER BASIS VON DICARBONSÄUREDIAMIDEN**
AMPHIPHILIC COMPOUNDS WITH AT LEAST TWO HYDROPHILIC AND AT LEAST TWO HYDROPHOBIC GROUPS BASED ON DICARBOXYCLIC ACID DIAMIDES
COMPOSES AMPHIPHILES COMPORTANT AU MOINS DEUX GROUPES HYDROPHILES ET AU MOINS DEUX GROUPES HYDROPHOBES A BASE DE DIAMIDES D'ACIDE DICARBOXYLIQUE

(30) Priorität: 20.08.1996 DE 19633497
(43) Veröffentlichungstag der Anmeldung: 07.07.1999
(73) Patentinhaber: SASOL Germany GmbH, 22297 Hamburg (DE)
(72) Erfinder: KWETKAT, Klaus, D-59192 Bergkamen (DE); JACOBS, Ulrike, D-45721 Haltern (DE); SCHOLZ, Silvia, D-45721 Haltern (DE)
(74) Vertreter: Schupfner, Gerhard D., Dr. Dipl.-Chem.
(86) Internationale Anmeldenummer: EP9703933
(87) Internationale Veröffentlichungsnummer: WO9807689

(56) Entgegenhaltungen:
- WO-A-96/25393
- FR-A- 1 464 243
- US-A- 2 028 091
- US-A- 2 192 906
- US-A- 3 246 023
- CHEMICAL ABSTRACTS, vol. 122, no. 22, 29.Mai 1995 Columbus, Ohio, US; abstract no. 268708, XP002046203 & JP 07 003 287 A (LION CORP) 6.Oktober 1995
- CHEMICAL ABSTRACTS, vol. 98, no. 12, 21.März 1983 Columbus, Ohio, US; abstract no. 98705, XP002046204 & P.E. KASHLINSKAYA ET AL: KOLLOIDN. ZH., Bd. 44, Nr. 6, 1982, Seiten 1170-1173,
- CHEMICAL ABSTRACTS, vol. 107, no. 22, 30.November 1987 Columbus, Ohio, US; abstract no. 200971, XP002046205 & L.M. KUDRYASHOVA ET AL: ZH. ANAL. KHIM., Bd. 42, Nr. 6, 1987, Seiten 1036-1040,

## Beschreibung

Die Erfindung betrifft amphiphile Verbindungen mit mindestens zwei hydrophilen und mindestens zwei hydrophoben Gruppen auf der Basis von Dicarbonsäurediamiden.

Als amphiphile Substanzen sind eine große Vielfalt an anionischen, kationischen, nichtionischen und zwitterionischen Verbindungen bekannt. Die weitaus meisten dieser Substanzen bestehen aus einer hydrophilen Kopfgruppe und wenigstens einem hydrophoben Teil.

Bei den amphiphilen Substanzen gibt es aus ökologischen Gründen, z. B. bezüglich der Verringerung des Verpackungs- und Transportaufwandes, die Notwendigkeit, immer größere Wirkung pro Masse an eingesetzter Substanz zu erzielen. Da eine Optimierung durch Mischung von amphiphilen Substanzen nur sehr begrenzt weiterführt, sind neue amphiphile Substanzen mit einem höheren Wirkungsgrad erforderlich. Es müssen daher insbesondere Stoffe mit niedrigeren kritischen Micellbildungskonzentrationen und/oder niedrigeren Ober- und Grenzflächenspannungen gefunden werden, um die Einsatzmengen an Wirksubstanz deutlich reduzieren zu können.

Erste Lösungsansätze in dieser Richtung durch Verdoppelung eines Teils der Struktur (hydrophile Kopfgruppe, hydrophobe Gruppe) sind bereits bekannt. So können kationische grenzflächenaktive Verbindungen durch die Addition von langkettigen Alkylhalogeniden an permethylierte Alkylendiamine erhalten werden [R. Zana, M. Benrraou, R. Rueff, Langmuir, 7 (1991) 1072; R. Zana, Y. Talmon, Nature, 362 (1993) 228; E. Alami, G Beinert, P. Marie, R. Zana, Langmuir, 9 (1993) 1465].

Anionische grenzflächenaktive Verbindungen mit wenigstens zwei hydrophilen und wenigstens zwei hydrophoben Gruppen sind auf der Basis von Diglycidylethern hergestellt worden (US 5 160 450, JP 01 304 033, JP 4 124 165). Diglycidylether gelten jedoch als toxikologisch bedenklich und sind recht teuer. Darüber hinaus wird für ihre Herstellung Epichlorhydrin verwendet, was zu großen Mengen an Reststoffen führt, so daß diese Verbindungen unter ökotoxikologischen wie auch ökonomischen Gesichtspunkten nicht mehr zeitgemäß sind.

Eine Vielzahl von Estern von α-Sulfocarbonsäuren und Polyalkylenoxidglykolethern, unter denen sich auch amphiphile Verbindungen befinden, werden in US-A-3 246 023 beschrieben. Den genannten Verbindungen werden nahezu Universaleigenschaften zugeschrieben. So sollen sie sich unter anderem als Emulgatoren für Insektizide, Pflanzenschutzmittel und Herbizide, als Additive für Petroprodukte und Gummilatices, als Schauminhibitoren, als Hilfsmittel beim Straßenbau, bei der Beton- und Papierherstellung, als Mercerisierungshilfsmittel, als Korrosionsinhibitoren, als Hilfsmittel bei der Herstellung kosmetischer Formulierungen und allgemein auch als Reinigungsmittel in hartem Wasser eignen.

Es bestand daher die Aufgabe, amphiphile Verbindungen aufzufinden, die wenigstens zwei hydrophile und wenigstens zwei hydrophobe Gruppen aufweisen, wobei die amphiphilen Verbindungen einen sehr hohen Wirkungsgrad, bezogen auf die Einsatzmenge, haben, und die darüber hinaus aus technisch leicht verfügbaren Rohstoffen ohne großen Anfall von unerwünschten Nebenprodukten hergestellt werden können.

Die Aufgabe wird erfindungsgemäß durch amphiphile Dicarbonsäurediamide, deren Grundkörper aus Dicarbonsäuren bzw. deren Estern und Alkylaminen hergestellt werden können, gelöst. Die entsprechenden Amide können sulfoniert und anschließend neutralisiert werden.

Bei den erfindungsgemäßen amphiphilen Verbindungen handelt es sich um Verbindungen der allgemeinen Formel I 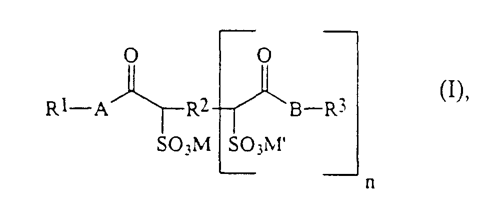 wobei R¹, R² und R³ in der Formel I die im folgenden beschriebenen Bedeutungen haben: R¹ und R³ stehen unabhängig voneinander für einen unverzweigten oder verzweigten, gesättigten oder ungesättigten Kohlenwasserstoffrest mit 8 bis 22 Kohlenstoffatomen.

Es seien als Substituenten R¹ und R³ im einzelnen die Reste n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Henicosyl, n-Docosyl und ihre verzweigtkettigen Isomeren sowie die entsprechenden einfach, zweifach oder dreifach ungesättigten Reste genannt.

Der Spacer R² bedeutet im einzelnen
◆ unverzweigte oder verzweigte Alkylenketten der Formel II

   -CₐH₂ₐ- (II)

   mit a = 2 bis 18, vorzugsweise a = 2 bis 6;
◆ Alicyclen gemäß der Formel IV

   -C_{f}H_{2f}-cyclo C₆H₁₀-C_{g}H_{2g}- (IV)

   mit f und g gleich unabhängig voneinander je 1 bis 6,
◆ gegebenenfalls substituierte Aromaten gemäß der Formel VI

   -CₕH₂ₕ-C₆R₄-(CᵢH₂ᵢ-C₆R₄)ⱼ-CⱼH₂ⱼ- (VI)

   oder gemäß der Formel VII

   -CₕH₂ₕ-C₁₀R₆-CⱼH₂ⱼ- (VII)

   mit h und j gleich unabhängig voneinander je 0 bis 8 und i = 0 bis 8 und mit R gleich unabhängig voneinander jeweils H oder C₁- bis C₆-Alkyl.

n in der Formel I steht für die Zahl 1.
R² kann weiterhin einem Methyl- oder Ethylester einer Polycarbonsäure (MW 500 bis 100 000, vorzugsweise 500 bis 1 000) entstammen, deren α-Kohlenstoffatome zwischen 0 und 100 %, bevorzugt zwischen 10 und 60 % sulfoniert worden sind; in diesem Fall liegt n zwischen 1 und 25 000.
M und M' stehen für Alkali-, Ammonium-, Alkanolammonium oder ½ Erdalkaliion.

A und B bedeuten unabhängig voneinander -NH-, -NR⁴- (mit R⁴ = Methyl, Ethyl, Propyl, Butyl oder Methoxyethyl, Methoxypropyl, Ethoxypropyl), -N(R⁵)-O(C₂H₄O)_{α}(C₃H₆O)_{β}- ( mit α = 0 bis 20, bevorzugt 0 bis 10, und β = 0 bis 20, bevorzugt 0 bis 10, α und β können nicht gleichzeitig 0 sein, und R⁵ gleich R⁴ oder gleich -O(C₂H₄O)_{α}(C₃H₆O)_{β}- H, mit den vorgenannten Bedingungen),oder -C(O)N(R⁵)-O(C₂H₄O)_{α}(C₃H₆O)_{β}- mit den vorgenannten Bedingungen.

Die erfindungsgemäßen amphiphilen Verbindungen zeichnen sich meist durch extrem niedrige kritische Micellbildungskonzentrationen (CMC) und sehr niedrige Oberflächen- und Grenzflächenspannungen (z. B. gegen Paraffin) aus, was auf ihre besondere Struktur - wenigstens zwei hydrophile Gruppen und wenigstens zwei hydrophobe Gruppen - zurückgeführt werden muß.

Darüber hinaus weisen die meisten von ihnen ein recht hohes hydrophiles Suspendiervermögen auf, das etwa auf halbem Wege zwischen dem konventioneller Tenside und dem des Pentanatriumtripolyphosphats liegt Einige dieser Verbindungen sind extrem schnelle Netzmittel. In der deutschen Offenlegungsschrift DE 39 32 492 werden Salze von sulfonierten Estern ungesättigter Dicarbonsäuren mit ungesättigten Fettalkoholen beschrieben. Diese Verbindungen weisen jedoch Strukturen auf, die von denen der erfindungsgemäßen Verbindungen völlig verschieden sind, da in DE 39 32 492 SO₃ ausschließlich an Doppelbindungen addiert wird.

Die amphiphilen Verbindungen gemäß dieser Erfindung eignen sich insbesondere als Emulgatoren, Demulgatoren, Detergenzien, Dispergatoren und Hydrotropica in Industrie und Haushalt, beispielsweise auf den Gebieten Metallbearbeitung, Erzgewinnung, Oberflächenveredelung, Waschen und Reinigen, Kosmetik, Medizin und Nahrungsmittelverarbeitung und -zubereitung.

Hierbei können sie mit allen gängigen anionischen, nichtionischen, kationischen und ampholytischen grenzflächenaktiven Substanzen kombiniert werden. Als Beispiele für nichtionische grenzflächenaktive Substanzen, die für eine Kombination eingesetzt werden können, seien Fettsäureglyceride, Fettsäurepolyglyceride, Fettsäureester, Ethoxylate höherer Alkohole,

Polyoxyethylenfettsäureglyceride, Polyoxyethylenpropylenglykolfettsäureester, Polyoxyethylensorbitanfettsäureester, Polyoxyethylen-Rhizinusöl- oder gehärtete Rhizinusöl-Derivate, Polyoxyethylenlanolinderivate, Polyoxyethylenfettsäureamide, Polyoxyethylenalkylamine, Alkanolamine, Alkylaminoxide, Derivate von Eiweißhydrolysaten, Hydroxymischether, Alkylpolyglycoside und Alkylglucamide genannt.

Als Beispiele für anionische grenzflächenaktive Substanzen, die für Kombinationen eingesetzt werden können, seien Seifen, Ethercarbonsäuren und deren Salze, Alkylsulfonate, α-Olefinsulfonate, Sulfonate höherer Fettsäureester, höhere Alkoholsulfate, Alkoholethersulfate, Hydroxymischethersulfate, Alkanoyl- und Alkenoylsarcosinate, Salze von Phosphatestern, Tauride, Isethionate, lineare Alkylbenzolsulfonate, Cumolsulfonat, Alkylarylsulfonate, Sulfate der Polyoxyethylenfettsäureamide und Salze von Acylaminosäuren genannt.

Als Beispiele für kationische gängige grenzflächenaktive Substanzen, die für Kombinationen eingesetzt werden können, seien Alkyltrimethylammoniumsalze, Dialkyldimethylammoniumsalze, Alkyldimethylbenzylammoniumsalze, Alkylpyridiniumsalze, Alkylisochinoliniumsalze, Benzethoniumchloride und kationische Acylaminosäurederivate genannt.

Als Beispiele für ampholytische grenzflächenaktive Substanzen, die für Kombinationen eingesetzt werden können, seien Aminosäuren, Betaine, Sulfobetaine, Imidazolinderivate, Sojaöllipide und Lecithin genannt.

Daruber hinaus können die erfindungseemäßen amphiphilen Verbindungen auch für sich miteinander kombiniert werden

Den erfindungsgemäßen amphiphilen Verbindungen können ebenfalls gängige Additive zugesetzt werden. Solche Additive werden speziell für eine Formulierung ausgewählt und umfassen üblicherweise anorganische Salze, wie Natriumchlorid und -sulfat, sowie Builder, Hydrotropica, UV-Absorber, Weichmacher, Chelatbildner, Viskositätsmodifizierer, Riechstoffe und Enzyme.

Die obengenannten Verbindungen lassen sich durch Amidierung von Dicarbonsäuren oder deren Ester mit Alkylaminen und durch die wenigstens doppelte Sulfonierung der Dicarbonsäuredialkylamide herstellen. Es sind beide Wege gangbar, sowohl, bevorzugt, die Sulfonierung von Dicarbonsäurealkylester (Alkylrest mit 1 bis 6 C-Atomen) mit anschließender Amidierung mit längeren Alkylaminopolyalkoxylaten oder Alkylaminen, als auch die Sulfonierung des bereits fertigen Grundgerüstes. Mit wäßrigen Alkali- oder Erdalkalihydroxiden oder wäßrigem Ammoniak oder Alkanolaminen wird neutralisiert. Bei Bedarf werden die Produkte in Substanz oder, bevorzugt, in wäßriger Lösung mit Wasserstoffperoxid (0,1 bis 2,0 %, bezogen auf Feststoff) gebleicht.

## Patentansprüche

1. Amphiphile Verbindungen der allgemeinen Formel I 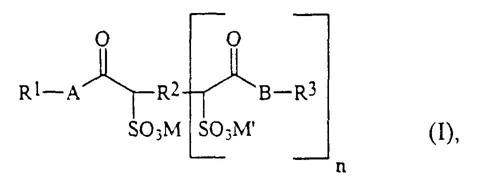 in der R¹ und R³ unabhängig voneinander einen unverzweigten oder verzweigten, gesättigten oder ungesättigten Kohlenwasserstoffrest mit 8 bis 22 Kohlenstoffatomen,
R²
◆ eine unverzweigte oder verzweigte Alkylenkette der Formel II
-CₐH₂ₐ- (II)
mit a = 2 bis 18, vorzugsweise a = 2 bis 6,
◆ einen Alicyclus gemäß der Formel IV
-C_{f}H_{2f}-cyclo C₆H₁₀-C_{g}H_{2g} (IV)
mit fund g gleich unabhängig voneinander je 1 bis 6
oder einen
◆ unsubstituierten oder substituierten Aromaten gemäß der Formel VI
-CₕH₂ₕ-C₆R₄-(CᵢH₂ᵢ-C₆R₄)ⱼ-CⱼH₂ⱼ- (VI)
oder gemäß der Formel VII
-CₕH₂ₕ-C₁₀R₆-CⱼH₂ⱼ- (VII)
mit h und j gleich unabhängig voneinander je 0 bis 8 und i = 0 bis 8 und mit R gleich unabhängig voneinander jeweils H oder C₁- bis C₆-Alkyl
bedeutet
oder einem Methyl- oder Ethylester einer Polycarbonsäure (MW 500 bis 100 000) entstammt, deren α-Kohlenstoffatome zwischen 0 und 100 %, bevorzugt zwischen 10 und 60 % sulfoniert worden sind,
M und M' für ein Alkali-, Ammonium-, Alkanolammonium- oder ½ Erdalkaliion steht,
A und B unabhängig voneinander -NH-, -NR⁴- (mit R⁴ = Methyl, Ethyl, Propyl, Butyl oder Methoxyethyl, Methoxypropyl, Ethoxypropyl), -N(R⁵)-O(C₂H₄O)_{α}(C₃H₆O)_{β}- ( mit α = 0 bis 20 und β = 0 bis 20, wobei α und β nicht gleichzeitig 0 sind und R⁵ gleich R⁴ oder gleich -O(C₂H₄O)_{α}(C₃H₆O)_{β}-H, mit den vorgenannten Bedingungen), oder -C(O)N(R⁵)-O(C₂H₄O)_{α}(C₃H₆O)_{β}- bedeuten
und n für die Zahl 1 steht, oder im Falle der Polycarbosäure zwischen 1 und 25 000 liegt.

2. Verwendung der amphiphilen Verbindungen nach Anspruch 1 als Emulgator.

3. Verwendung der amphiphilen Verbindungen nach Anspruch 1 als Demulgator.

4. Verwendung der amphiphilen Verbindungen nach Anspruch 1 als Hilfsmittel bei der Metallbearbeitung, Erzgewinnung oder Oberflächenveredelung.

5. Verwendung der amphiphilen Verbindungen nach Anspruch 1 als Textilhilfsmittel oder für das Reinigen und Waschen von Textilien.

6. Verwendung der amphiphilen Verbindungen nach Anspruch 1 für das Reinigen von harten Oberflächen.

7. Verwendung der amphiphilen Verbindungen nach Anspruch 1 für das Reinigen und Waschen von Haut und Haar.

## Claims

1. Amphiphilic compounds having the general formula I 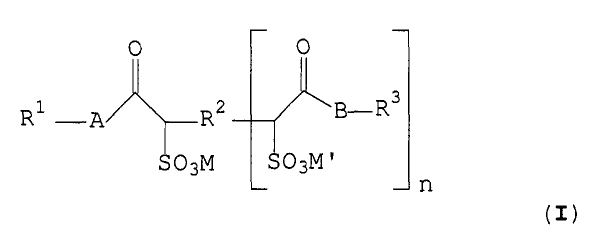 wherein **R**^{**1**} and **R**^{**3**}**,** independently of one another, are an unbranched or branched, saturated or unsaturated hydrocarbon radical with 8 to 22 carbon atoms,
**R**^{**2**} is
◆ an unbranched or branched alkylene chain of formula II
-CₐH₂ₐ- (**II**)
where **a** = 2 to 18, preferably a = 2 to 6,
◆ an alicyclic compound of formula IV
-C_{f}H_{2f}-cycloC₆H₁₀-C_{g}H_{2g} (**IV**)
where **f** and **g,** independently of one another, is 1 to 6 each,
or an
◆ unsubstituted or substituted aromatic compound of formula VI
-CₕH₂ₕ-C₆R₄-(CᵢH₂ᵢ-C₆R₄)ⱼ-CⱼH₂ⱼ- (**VI**)
or of formula VII
-CₕH₂ₕ-C₁₀R₆-CⱼH₂ⱼ- (**VII**)
where each **h** and **j**, independently of one another, are equal to from 0 to 8 and **i** is equal to from 0 to 8 and each **R,** independently of one another, is equal to H or C₁- to C₆-alkyl,
or is derived from a methyl or ethyl ester of a polycarboxylic acid (Mw 500 to 100,000) the α-carbon atoms of which have a sulfonation degree of from 0 to 100 %, preferably 10 to 60 %,
**M** and **M'** represent an alkali, ammonium, alkanol ammonium, or ½ alkaline earth ion,
**A** and **B,** independently of one another, are -NH-, -NR⁴- (where **R**^{**4**} is equal to methyl, ethyl, propyl, butyl, or methoxyethyl, methoxypropyl, ethoxypropyl), -N(R⁵)-(C₂H₄O)_{α}(C₃H₆O)_{β}- (where α is equal to from 0 to 20 and β is equal to from 0 to 20, where α and β are not simultaneously 0, and **R**^{**5**} is equal to **R**^{**4**} or -O(C₂H₄O)_{α}(C₃H₆O)_{β}-H under the conditions set forth hereinabove), or-C(O)N(R⁵)-O(C₂H₄O)_{α}(C₃H₆O)_{β}-,
and **n** is the number 1 or, in the case of polycarboxylic acid, is a number from 1 to 25,000.

2. Use of the amphiphilic compounds of claim 1 as emulsifiers.

3. Use of the amphiphilic compounds of claim 1 as demulsifiers.

4. Use of the amphiphilic compounds of claim 1 as auxiliaries in metal working, ore mining, or surface finishing.

5. Use of the amphiphilic compounds of claim 1 as textile auxiliaries or for cleaning and washing textiles.

6. Use of the amphiphilic compounds of claim 1 for cleaning hard surfaces.

7. Use of the amphiphilic compounds of claim 1 for cleaning and washing skin and hair.

## Revendications

1. Composés amphiphiles anioniques de formule générale I
où
R¹ et R³ représentent, indépendamment l'un de l'autre, un radical hydrocarboné non ramifié ou ramifié, saturé ou insaturé comprenant 8 à 22 atomes de carbone
R² représente
* une chaîne alkylène non ramifiée ou ramifiée de formule II
-CₐH₂ₐ- (II)
avec a = 2 à 18, de préférence a = 2 à 6,
* un alicycle selon la formule IV
-C_{f}H_{2f}-cyclo C₆H₁₀-C_{g}H_{2g}- (IV)
avec f et g, chacun égal à 1 jusqu'à 6 indépendamment l'un de l'autre
ou
* un aromate le cas échéant substitué ou non substitué selon la formule VI
-CₕH₂ₕ-C₆R₄-(CᵢH₂ᵢ-C₆R₄)ⱼ-CⱼH₂ⱼ- (VI)
ou selon la formule VII
-CₕH₂ₕ-C₁₀R₆-CⱼH₂ⱼ - (VII)
avec h, j, chacun égal à 0 à 8 indépendamment l'un de l'autre et avec i = 0 à 8 et avec R à chaque fois égal à H ou à un alkyle en C₁ à C₆ indépendamment l'un de l'autre
ou provient d'un ester méthylique ou d'un ester éthylique d'un acide polycarboxylique (PM 500 à 100000), dont les atomes de carbone en α sont sulfonés entre 0% et 100%, de préférence entre 10% et 60%
M et M' représentent un ion alcalin, un ion ammonium, un ion alcanolammonium ou 1/2 ion alcalino-terreux,
A et B signifient indépendamment l'un de l'autre -NH-, -NR⁴- (avec R⁴ = méthyle, éthyle, propyle, butyle ou méthoxyéthyle, méthoxypropyle, éthoxypropyle), -N(R⁵)-O(C₂H₄O)_{α}(C₃H₆O)_{β}- (avec α = 0 à 20, de préférence 0 à 10, et β = 0 à 20, de préférence 0 à 10, α et β ne pouvant être simultanément égaux à 0 et R⁵ étant identique à R⁴ ou à -O(C₂H₄O)_{α}(C₃H₆O)_{β}-H avec les conditions susmentionnées) ou -C(O)N(R⁵)-O(C₂H₄O)_{α}(C₃H₆O)_{β}-
et n représente le nombre 1 ou, dans le cas de l'acide polycarboxylique, est situé entre 1 et 25000.

2. Utilisation des composés amphiphiles selon la revendication 1 comme émulsifiant.

3. Utilisation des composés amphiphiles selon la revendication 1 comme désémulsifiant.

4. Utilisation des composés amphiphiles selon la revendication 1 comme adjuvant pour le traitement des métaux, l'extraction des minerais ou l'amélioration des surfaces.

5. Utilisation des composés amphiphiles selon la revendication 1 comme adjuvant pour textiles ou pour le lavage et le nettoyage de textiles.

6. Utilisation des composés amphiphiles selon la revendication 1 pour le nettoyage de surfaces dures.

7. Utilisation des composés amphiphiles selon la revendication 1 pour le nettoyage et le lavage de la peau et des cheveux.
